# EUROPEAN PATENT APPLICATION

(11) **EP 4 559 377 A1**
(43) Date of publication of application: **28.05.2025**
(21) Application number: 23876139.9
(22) Date of filing: 25.04.2023
(51) Int. Cl.: A61B 1/005

(54) **CONTROLLABLE BENDING MECHANISM FOR ENDOSCOPE, AND ENDOSCOPE**

(30) Priority: 10.10.2022 CN 202211234890
(71) Applicant: Anqing Medical Co., Ltd, Shanghai 201201 (CN)
(72) Inventor: YAN, Hang, Shanghai 201201 (CN); TANG, Wei, Shanghai 201201 (CN)
(74) Representative: Metida
(86) International application number: PCT/CN2023/090615
(87) International publication number: WO 2024/077928

(57) **Abstract**

The invention provides a controllable bending mechanism of an endoscope and the endoscope, including: a hollow tube, wherein a plurality of groups of gap sets are disposed on the hollow tube; the plurality of groups of gap sets extend along an axial direction of the hollow tube; each group of gap sets includes a first gap portion and a second gap portion, wherein the first gap portion is distributed in a first circumferential direction of the hollow tube; the second gap portion is distributed in a second circumferential direction of the hollow tube; the first gap portion includes two gaps; the length of each gap is equivalent to 1/2 of that of the circumference; the second gap portion includes two gaps; the length of each gap is equivalent to 1/2 of that of the circumference; along the circumferential direction of the hollow tube, the two gaps of the first gap portion and the two gaps of the second gap portion respectively have a relative position difference of a pre-set length; and the gap has an olive shape along the length of the gap. According to the technical solution of the invention, it has smooth steering, flexible rotation and sensitive bending.

## Description

### TECHNICAL FIELD

The invention relates to the technical field of endoscopes, and in particular to a controllable bending mechanism of an endoscope and the endoscope.

### BACKGROUND ART

It is well known to cut a plurality of tube sections on a hollow steel tube by a laser. Each tube section includes a connection unit which cooperates with a connection unit of an adjacent tube section to finally form a controllable bending portion at the distal end of an endoscope insertion tube (commonly known as "snake bone").

The bending mechanisms of endoscopes currently available on the market are divided into structures with and without pins.

In the structures with pins, the riveted pin structure can be made to a four-way bending snake bone with flexible rotation and good bending mechanical properties. However, as the radial dimension of the riveted pin is too large and not smooth, the manufacturing process is difficult and the cost is high.

In the structures without pins, the snake bone with different patterns cut on the hollow steel tube by the laser is made to have two-way bending, which does not have enough steering stability. If the snake bone is made to have four-way bending, it cannot meet the requirements of rotational flexibility, bending mechanical properties and structural strength.

### SUMMARY OF THE INVENTION

The invention provides a controllable bending mechanism of an endoscope and the endoscope, so as to solve the problems of the prior art that the steering smoothness, the rotational flexibility, the bending mechanical properties, the structural strength, etc. do not meet the requirements.

In order to solve the above technical problem, the invention is achieved by the following technical solutions.

According to a first aspect of the invention, a controllable bending mechanism of an endoscope is provided, including a hollow tube, wherein a plurality of groups of gap sets are disposed on the hollow tube; the plurality of groups of gap sets extend in sequence along an axial direction of the hollow tube;
each group of gap sets includes a first gap portion and a second gap portion, wherein the first gap portion is distributed in a first circumferential direction of the hollow tube; the second gap portion is distributed in a second circumferential direction of the hollow tube;
the first gap portion includes two gaps; the length of each gap is equivalent to 1/2 of that of the circumference, namely, the length of the gap is close to but less than half of the circumference;
the second gap portion includes two gaps; the length of each gap is equivalent to 1/2 of that of the circumference, namely, the length of the gap is close to but less than half of the circumference;
along the circumferential direction of the hollow tube, the two gaps of the first gap portion and the two gaps of the second gap portion respectively have a relative position difference of a pre-set length; and
the gap has an olive shape along the length of the gap.

Preferably, the gap is bent around an axial center of the axial direction by a preset arc length; and the positions where both sides of the gap are contacted are two parallel lines after being contacted.

Preferably, the gap is bent around an axial center of the axial direction by a preset arc length; and the positions where both sides of the gap are contacted are two parallel straight lines after being contacted.

Preferably, the preset arc length is equal to a gap width at a 1/2 length position of the gap.

Preferably, both ends of the gap are respectively provided with destressing incisions.

Preferably, the destressing incision is a scalloped incision, the arc of the scalloped incision facing outwards.

Preferably, the center of the scalloped incision is located in the corresponding circumferential direction.

Preferably, a portion of the scalloped incision facing towards the center of the scalloped incision is not cut, leaving an area support structure.

Preferably, along the circumferential direction of the hollow tube, the two gaps of the first gap portion and the two gaps of the second gap portion respectively have a relative position difference of a pre-set length; specifically, the two gaps of the first gap portion and the two gaps of the second gap portion respectively have a relative position difference of 1/4 of the circumference.

Preferably, the gap has a gap width greater than a predetermined gap width value.

Preferably, the axial spacing between the first gap portion and the second gap portion is less than a preset axial spacing value.

According to a second aspect of the invention, an endoscope is provided, including the controllable bending mechanism of the endoscope as described in any one of the above.

According to the controllable bending mechanism of the endoscope and the endoscope provided in the invention, the gap has an olive-shaped structure along the length direction of the gap, which can effectively increase the axial support and the radial bending softness and can improve the stability of rotation when ensuring a larger bending arc length or bending angle.

The controllable bending mechanism of the endoscope and the endoscope provided in the invention have a relative position difference of a pre-set length between two gaps of the first gap portion and two gaps of the second gap portion respectively, and are flexible in rotation and sensitive in bending. In addition, the mechanism of the invention is easy to manufacture, reliable in connection, not easy to wear, low in manufacturing cost and convenient in use.

In an alternative solution of the invention, the gap is bent around an axial center of the axial direction by a preset arc length; and the positions where both sides of the gap are contacted are two parallel straight lines after being contacted, which can play a guiding role to further enhance the steering stability and consistency of the snake bone.

In an alternative solution of the invention, the width of the olive-shaped gaps at both ends is relatively narrow. In order to improve the destressing effect at both ends, both ends of the gaps are provided with destressing incisions which are provided to reduce the stress.

In an alternative solution of the invention, the gap width of the gap is determined by the turning radius. By adjusting the gap width of the gap, the turning radius of the snake bone can be arbitrarily changed, and a variety of radius-variable bending snake bones can be made. The greater the gap width is, the smaller the turning radius is. The axial spacing between the two gap portions is determined by the turning stiffness. The turning softness of the snake bone can be adjusted by adjusting the axial spacing between the two gap portions. The smaller the axial spacing is, the softer the turning is.

### BRIEF DESCRIPTION OF THE DRAWINGS

In order to more clearly describe the technical solutions in the examples of the disclosure or the prior art, the drawings to be used in the description of the examples or the prior art will be briefly introduced below. It will be apparent to those skilled in the art that the drawings in the following description are only some of the disclosure, and that other drawings may be obtained from the drawings without any creative works.
Fig. 1 is a projection view of a partially hollow tube according to an example of the invention;
Fig. 2 is a circumferentially expanded view of a partially hollow tube according to an example of the invention;
Fig. 3 is an enlarged view of a set of gaps of Part A of Fig. 2; and
Fig. 4 is a circumferentially expanded view of a partially hollow tube according to another example of the invention;

### Description of Reference Numerals:

1-first gap portion,
11-gap,
12-gap;
2-second gap portion,
21-gap,
22-gap,
3-olive shape,
4-scalloped incision.

### DETAILED DESCRIPTION OF THE INVENTION

The technical solutions in the examples of the invention will be described clearly and completely in conjunction with the accompanying drawings in the examples of the invention. Obviously, the described examples are only part of the examples of the invention, rather than all of the examples. Based on the examples in the invention, all other examples obtained by a person skilled in the art without involving any inventive effort are within the scope of protection of the invention.

In the description of the invention, it should be understood that the directional or positional relationships indicated by the terms "upper", "lower", "upper end", "lower end", "lower surface", "upper surface" and the like are based on the directional or positional relationships shown in the drawings. It is merely for the purpose of describing the disclosure and simplifying the invention, and is not intended to indicate or imply that a particular orientation, configuration and operation of the referenced device or element is required and should not be construed as limiting the invention.

In the description of the invention, the terms "first" and "second" are used for descriptive purposes only and are not to be construed as indicating or implying relative importance or implicitly indicating the number of technical features indicated. Thus, a feature defined by "first" or "second" may explicitly or implicitly includes one or more such features.

In the description of the invention, "a plurality of" means multiple, e.g., two, three, four, etc. unless specifically and specifically limited otherwise.

In the description of the invention, unless expressly stated or limited otherwise, the terms "connected" and the like are to be interpreted broadly, e.g., either connected fixedly or detachably, or integrated. It may be a mechanical connection or an electrical connection. It may be a direct connection or indirect connection by an intermediary. It may be a communication between two elements, or may be in an interactive relationship between two elements. The specific meaning of the above terms in this invention will be understood in specific circumstances by those of ordinary skill in the art.

Hereinafter, technical solutions of the invention will be described in detail with reference to specific examples. The following specific examples may be combined with one another, and the same or similar concepts or processes may not be repeated in some examples.

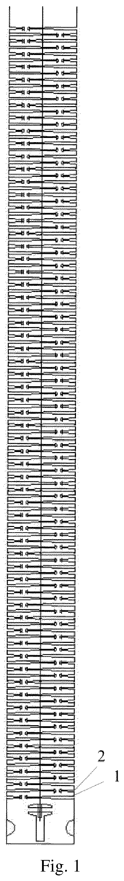

In an example, an endoscope controllable bending mechanism is provided, including a hollow tube, wherein a plurality of groups of gap sets are disposed on the hollow tube, and the plurality of groups of gap sets extend in sequence along the axial direction of the hollow tube. See Figs. 1, 2 and 3.

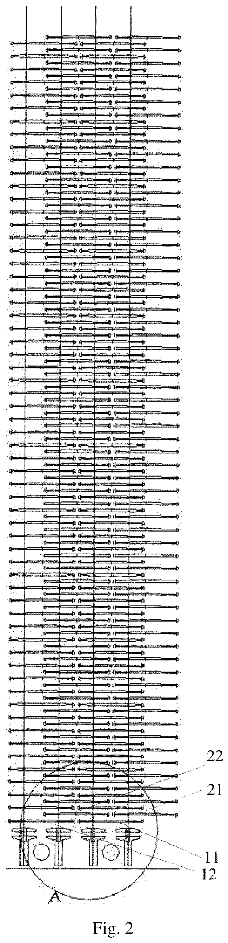

Here, each group of gap sets includes a first gap portion 1 and a second gap portion 2. The first gap portion 1 is distributed in a first circumferential direction of the hollow tube. The second gap portion 2 is distributed in a second circumferential direction of the hollow tube. See Figs. 1 and 2.

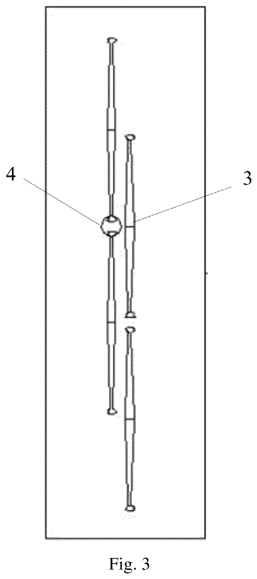

The first gap portion includes two gaps 11, 12. The length of each gap is equivalent to 1/2 of the circumference, namely, the length of the gap is close to but less than half of the circumference. The second gap portion includes two gaps 21, 22, each gap having a length corresponding to 1/2 of the circumference, i.e., the length of the gap is close to but less than half of the circumference. Along the circumferential direction of the hollow tube, two gaps of the first gap portion 1 have a relative position difference of a preset length from two gaps of the second gap portion 2, respectively. See Fig. 2. The gap has an olive shape 3 along the length of the gap. See Fig. 3.

In the controllable bending mechanism of the endoscope of the above-mentioned example, the gap is provided in an olive shape, so that the smoothness of rotation can be improved. The specific reasons are as follows. The gaps in the prior art are mostly rectangular gaps, namely, the gap is a rectangular gap. When the hollow tube is bent until both sides of the gap are closed, two points must first contact, so that the axial support is insufficient, resulting in unstable rotation. The gap of the invention has an olive shape. When the hollow tube is bent to close both sides of the gap, two lines are contacted, which is smoother than the contact of two points, so as to improve the axial support and thus improve the smooth rotation.

The controllable bending mechanism of the endoscope of the invention has a symmetrical structure, and has no wiring groove of the traction wire formed by special laser cutting, which can effectively increase the number of bending joints and increase the bending angle. The wiring groove of the traction wire may be arbitrarily set at any tube section position at the position shown in the figure, and may be formed by subsequent stamping. The 1/2 circumferential symmetric distribution of the traction wire may achieve two-way controllable bending. The 1/4 circumferential symmetric distribution may achieve four-way controllable bending.

In an example, the gap is bent around the axial center by a preset arc length. After the both sides of the gap are contacted, the positions where the both sides of the gap are contacted are two parallel lines.

In an example, the gap is bent around an axial center of the axial direction by a preset arc length; and the positions where both sides of the gap are contacted are two parallel straight lines after being contacted, which can play a guiding role to further enhance the steering stability and consistency of the snake bone. Of course, in various examples, the position at which the both sides of the gap are contacted is not necessarily a strictly straight line, but may be two parallel arcs.

In an example, the preset arc length is equal to the gap width at the 1/2 length position of the gap.

In an example, along the circumferential direction of the hollow tube, the two gaps of the first gap portion and the two gaps of the second gap portion respectively have a relative position difference of a pre-set length. Specifically, the two gaps of the first gap portion have a relative position difference of 1/4 of the circumference from the two gaps of the second gap portion, respectively. See Fig. 2.

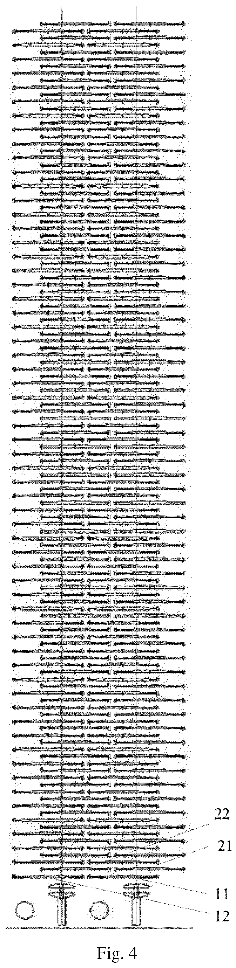

In an example, the relative position difference between the gap of the first gap portion and the gap of the second gap portion can also be adjusted according to needs. With reference to Fig. 4, when the snake bone is bent in two directions, the phase position difference may be adjusted to be less than 1/4 of the circumference, which can not only further ensure the stability and consistency of steering when rotating in two directions, but also further preserve the softness of steering. It can increase the passage of the endoscope when inserting.

In an example, since both ends of the olive-shaped gap are relatively narrow, in order to reduce stress, both ends of the gap are provided with destressing incisions.

In an example, the destressing incision may be a scalloped incision with the arc of the scalloped incision facing outwards. See Fig. 3. The scalloped incision provides better stress reduction.

In an example, the center of the scalloped incision is located in the corresponding circumferential direction.

In an example, the portion of the scalloped incision facing towards the center of the scalloped incision is an uncut portion, leaving an area support structure. See Fig. 3.

In an example, the width of the gap is determined by the turning radius. The gap width may be greater than a preset gap width value. The greater the gap width, the smaller the turning radius, the more flexible the turning, and vice versa.

In an example, the axial spacing between the first gap portion and the second gap portion is determined by the turning stiffness. The pre-set axial spacing may be less than the pre-set axial spacing value. The smaller the axial spacing, the softer the bend, and conversely, the harder the bend.

In different examples, different gap widths and axial spacing can be designed according to different requirements of turning radius. In the design, the following aspects need to be considered: 1, will it cause fatigue during elastic deformation. 2, a certain wall thickness must be maintained in order to maintain a certain support. 3, is the turning length is a turning semicircumference or a turning full-circumference, which must be taken into account; 4, for the material, the elasticity of different materials is different.

In an example, the tip of the hollow tube can also be provided with an avoidance hole of a locking head end of the traction wire, which can effectively increase the inner diameter space utilization rate of the snake bone.

In an example, an endoscope is also provided, including the controllable bending mechanism of the endoscope as described in any one of the examples described above.

In the disclosure of this description, reference to the terms "an embodiment", "an example", and "a specific implementation process", "an instance", etc., means that specific features, structures, materials, or characteristics described in connection with the embodiment or example is included in at least one embodiment or example of the invention. In the present specification, schematic statement of the above terms does not necessarily refer to the same embodiment or example. Furthermore, the particular features, structures, materials, or characteristics described may be combined in any suitable manner in any one or more embodiments or examples.

Finally, it should be noted that each example above are only intended to illustrate the technical solution of the invention, but not to limit it. Although the invention has been described in detail with reference to the each foregoing example, those skilled in the art will appreciate that the technical solutions of the each above-mentioned example can still be modified, or some of the technical features thereof can be equivalently substituted. Such modifications and substitutions will not cause the essence of the corresponding technical solutions to depart from the scope of the examples of the invention.

## Claims

1. A controllable bending mechanism of an endoscope, comprising a hollow tube, wherein a plurality of groups of gap sets are disposed on the hollow tube; the plurality of groups of gap sets extend in sequence along an axial direction of the hollow tube;
each group of gap sets comprises a first gap portion and a second gap portion, wherein the first gap portion is distributed in a first circumferential direction of the hollow tube; the second gap portion is distributed in a second circumferential direction of the hollow tube;
the first gap portion comprises two gaps; the length of each gap is equivalent to 1/2 of that of the circumference;
the second gap portion comprises two gaps; the length of each gap is equivalent to 1/2 of that of the circumference;
along the circumferential direction of the hollow tube, the two gaps of the first gap portion and the two gaps of the second gap portion respectively have a relative position difference of a pre-set length; and
the gap has an olive shape along the length of the gap.

2. The controllable bending mechanism of the endoscope according to claim 1, wherein the gap is bent around an axial center of the axial direction by a preset arc length; and the positions where both sides of the gap are contacted are two parallel lines after being contacted.

3. The controllable bending mechanism of the endoscope according to claim 2, wherein the preset arc length is equal to a gap width at a 1/2 length position of the gap.

4. The controllable bending mechanism of the endoscope according to claim 1, wherein both ends of the gap are respectively provided with destressing incisions.

5. The controllable bending mechanism of the endoscope according to claim 1, wherein the destressing incision is a scalloped incision, the arc of the scalloped incision facing outwards.

6. The controllable bending mechanism of the endoscope according to claim 5, wherein the center of the scalloped incision is located in the corresponding circumferential direction.

7. The controllable bending mechanism of the endoscope according to claim 6, wherein a portion of the scalloped incision facing towards the center of the scalloped incision is not cut.

8. The controllable bending mechanism of the endoscope according to claim 1, wherein along the circumferential direction of the hollow tube, the two gaps of the first gap portion and the two gaps of the second gap portion respectively have a relative position difference of a pre-set length; specifically, the two gaps of the first gap portion and the two gaps of the second gap portion respectively have a relative position difference of 1/4 of the circumference.

9. The controllable bending mechanism of the endoscope according to any one of claims 1 to 8, wherein the gap has a gap width greater than a predetermined gap width value.

10. The controllable bending mechanism of the endoscope according to any one of claims 1 to 8, wherein the axial spacing between the first gap portion and the second gap portion is less than a preset axial spacing value.

11. An endoscope, comprising the controllable bending mechanism of the endoscope according to any one of claims 1 to 10.
